# EUROPEAN PATENT SPECIFICATION

(11) **EP 1 558 298 B1**
(45) Date of publication and mention of the grant of the patent: **05.12.2007**
(21) Application number: 03759471.0
(22) Date of filing: 19.09.2003
(51) Int. Cl.: A61L 9/12, B60H 3/00, A01M 1/20, A61L 9/03

(54) **DEVICE FOR DIFFUSING VOLATILE SUBSTANCES TO AMBIENT AIR COMPRISING A WICK WITH PREDETERMINED POROSITY**
VORRICHTUNG ZUR DIFFUSION VON FLÜCHTIGEN SUBSTANZEN IN DIE UMGEBUNGSLUFT BEINHALTEND EINEN DOCHT MIT VORBESTIMMTER POROSITÄT
DISPOSITIF DE DIFFUSION DE SUBSTANCES VOLATILES DANS L'AIR AMBIANT, COMPRENANT UNE MECHE AVEC UNE POROSITE PREDETERMINEE

(30) Priority: 08.10.2002 US 266798
(43) Date of publication of application: 03.08.2005
(73) Proprietor: S. C. Johnson & Son, Inc., Racine, WI 53403 (US)
(72) Inventor: LAKATOS, Kara, L., Racine, WI 53406 (US); WEEKS, Ann, E., Racine, WI 53406 (US); VARANASI, Padma, P., Racine, WI 53402 (US)
(74) Representative: Ruschke, Hans Edvard
(86) International application number: PCT/US2003/030025
(87) International publication number: WO 2004/032984

(56) References cited:
- EP-A- 0 134 360
- WO-A-20/04032620
- FR-A- 2 587 622
- US-A- 3 587 968
- US-A- 5 909 845
- US-A1- 2002 136 886

## Description

### BACKGROUND OF THE INVENTION

### 1. Field of the Invention

The present invention relates to a device for transporting liquids, such as insect repellant, fragrances, or insecticides, from a reservoir to a surface exposed to the ambient air.

### 2. Description of the Related Art

Devices that release vapors into the ambient air are well-known in the art. Generally, the purpose of these devices is to deodorize or disinfect the ambient air, or to distribute toxins into the air to kill or repel unwanted pests, such as mosquitoes.

To achieve the goal of dispensing vapors into the air, a number of methods has been employed. For example, aerosol containers have been used to eject vapors into the air upon the activation of a trigger by the user. Other methods, however, utilize the evaporative properties of liquids, or other vaporizable materials, to cause vapors with desired properties to be distributed into the ambient air. One such evaporative method utilizes a wick to deliver a vaporizable liquid from a reservoir to a surface exposed to the ambient air. As the liquid reaches the exposed surface, the liquid is vaporized and dispersed into the ambient air. The exposed surface may be either the surface of the wick or the surface of another body in fluid communication with the wick.

Because such wick-based delivery systems require the exposure of a surface to the ambient air and a path for the vaporizable liquid to reach that exposed surface, careful designing of the device is necessary to prevent unwanted leakage of the liquid from the device. Moreover, since these liquids typically are intended to be dispersed only in their vaporized form, these liquids tend to consist of a high concentration of the active ingredient (e.g., fragrance or insecticide). Therefore, even a small amount of leakage can be bothersome to the consumer, and it is a goal of designers of wick-based delivery systems to minimize the possibility that any leakage of the liquid from the bottle will occur. Of course, it is also a desired goal of manufacturers of wick-based delivery systems to create a device that is simple and effective in operation, as well as simple and cost-effective to manufacture.

When wick-based delivery systems are accidentally overturned, either during packaging, shipping, or use by the consumer, spilling or leakage of the liquid can occur. Such leakage can occur through the wick itself, or through any other opening in the wick-based system. One particular source of leakage is through a vent-hole in the bottle. Vent-holes are common in wick-based delivery systems because they help maintain a constant release rate of the liquid into the ambient air by preventing the formation of a vacuum in the head-space of the bottle.

The wick transports the liquid to the surface of the wick by a principle called capillary action. In particular, the wick material contains numerous pores, and these pores act as capillaries, which cause the liquid to be drawn into them. As the liquid is drawn from the reservoir and transported up the porous wick, a vacuum is created in the head-space of the bottle. The formation of a vacuum in the head-space of the bottle decreases the rate that the liquid is wicked from the reservoir to the surface. Of course, this decrease in the wicking rate translates directly into a decrease in the release rate of the evaporated liquid to the ambient air.

In order to combat the formation of the vacuum in the head-space, conventional wick-based delivery systems contain a vent-hole in the vicinity of the head-space of the bottle. These vent-holes prevent the formation of a vacuum in the head-space of the bottle and, therefore, prevent the occurrence of a decrease in the release rate of the liquid to the ambient air.

As noted above, however, vent-holes are a source of leakage of the liquid from the reservoir during shipping and/or handling of the bottle by the consumer.

An example of a wick-based, controlled release device is described in U.S. Patent No. 4,915,301. This patent discloses a bottle for dispensing a liquid in vapor phase. More specifically, the bottle contains a liquid and that liquid is absorbed by a wick and conveyed to a porous body. The liquid then spreads through the porous body and reaches a microporous membrane which permits the liquid to be discharged as a vapor into the atmosphere. The membrane serves to enable emission of vapors of the liquid, while preventing passage of the liquid itself. Although the membrane helps prevent spillage of the liquid through the wick, this system requires a vent-hole in the head-space of the bottle, through which the liquid may spill or leak. This system also requires the manufacture and arrangement of three elements (i.e., the wick, porous body, and membrane) in order to transport the liquid from the reservoir to the ambient air.

U.S. Patent No. 4,419,326 discloses a vapor dispensing device in which at least one porous wick extends to transfer an evaporative agent from a container to a porous plastic element that is exposed to the ambient. However, this patent discloses that the vapor pressure in the head-space of the container should be maintained at least at the level of the ambient pressure. In order to achieve this, the patent suggests that a wicking element can be used to act as a vent-hole to relieve the build-up of pressure in the head-space of the container.

U.S. Patent No. 5,437,410 discloses a passive aromatic substance dispenser that does not require a vent-hole. However, the design of the dispenser is inverted and, therefore, utilizes the influence of gravity to draw the aromatic substance through the dispenser.

Another inverted device is disclosed in U.S. Patent No. 6,109,539. This patent discloses a device that comprises a housing, a volatile substance, a porous plug, and an interior region. The housing is constructed of a material that is substantially permeable to ambient air, but substantially impermeable to the volatile substance contained within the housing.

Other patents have disclosed using stoppers that allow the passage of air, thus creating an effective vent-hole. For example, U.S. Patent No. 4,413,779 relates to a vapor dispensing device in which at least one rigid porous wick extends to transfer a liquid from the container to a porous plastic element from the surface of which dispersion of the agent will occur by means of evaporation. Pressure control in the head-space of the container is achieved by permitting sufficient air to pass through a stopper that is positioned between the reservoir and the plastic element. U.S. Patent No. 4,413,779 also discloses that a volatile liquid that produces a vapor pressure within the container equal to or exceeding the ambient pressure can be used to counter the effects of the formation of a vacuum in the head-space.

Another attempt to prevent leakage of the liquid in a wick-based delivery system is disclosed in U.S. Patent No. 3,550,853. This patent relates to a dispenser unit with a barrier or wad of porous resilient plastic foam through which a saturated vapor must pass as it is emitted into the ambient air. The density of the plastic foam material is selected so that it permits the passage of vapor from a storage chamber, but occludes the passage of liquid. This helps prevent leakage when the device is overturned. However, this design calls for both a wick and a plastic foam material in order to disperse the vaporizable material from the liquid to the ambient air.

Document FR-A-2587622 on which the pre-characterizing part of claim 1 is based discloses a wick-based delivery system in which the wick has a bulbous top that completely fills the neck of the bottle and presents a large area for evaporation. The pore size is in the range 0.8 microns to 20.00 microns. Specific examples give pore sizes of 75, 50, 500 and 5000 microns. Other examples of documents showing a large evaporation surface are US 3,587,968 and EP-A-0 134 360.

The invention seeks to provide a delivery system that does not require a vent hole to relieve the vacuum in the headspace above the liquid, yet at the same time has good evaporation properties.

### SUMMARY OF THE INVENTION

The present invention provides a device as defined in claim 1 below.

A better understanding of these and other features and advantages of the invention may be had by reference to the drawings and to the accompanying description, in which preferred embodiments of the invention are illustrated and described.

### BRIEF DESCRIPTION OF THE DRAWINGS

Figure 1 shows an exploded view of a wick-based delivery system according to a first preferred embodiment of the present invention.

Figure 2 shows a side view of a wick according to another preferred embodiment of the present invention.

Figure 3 shows a view of a wick-based delivery system according to the present invention being utilized in conjunction with an optional electric plug-in heater.

Throughout the figures, like or corresponding reference numerals have been used for like or corresponding parts.

### DETAILED DESCRIPTION OF THE INVENTION

The present invention relates to a wick-based delivery system for transporting a liquid from a reservoir to a surface that is exposed to the ambient air. In its simplest form, the system of the invention comprises a container for holding a liquid, and a wick for transporting the liquid from the container to an upper surface of the wick.

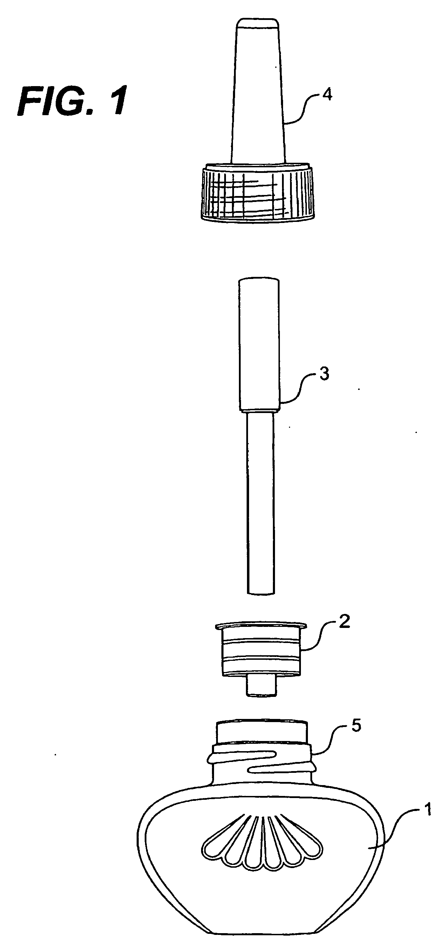

The container can be formed in a variety of shapes. In Figure 1, for example, the container is a bottle **1** of conventional shape. A vaporizable liquid is added to the bottle **1**. (The level of the liquid is not shown in the bottle **1**.) A wick **3** should be shaped so that it fits snugly into a neck **5** of the bottle **1**. It is preferable to use a neck closure **2**, such as that shown in Figure 1, to hold the wick **3** in place and to prevent leakage around the neck **5** of the bottle **1**. The fit between the neck closure **2** and the bottle **1** should be tight enough to prevent leakage of the liquid from the bottle **1**. Likewise, the fit between the neck closure **2** and the wick **3** should be sufficiently tight to prevent leakage of the liquid from the bottle **1**.

In addition, the neck **5** of the bottle **1** can be shaped so that a cover **4** can be securely fastened over the wick **3** and neck closure **2**. For example, the outer neck **5** of the bottle **1** may be threaded so that a cover **4** can be screwed on top of the bottle **1** when the device is not in use.

The bottle **1** and the neck closure **2** can be made of any suitable material that is leakproof. Of course, the size of the opening in the bottle **1** and the size of the neck closure **2** are dependent upon each other and upon the size of the wick **3** that is to be used with the device.

As described above, when the liquid is drawn from the bottle **1** and transported up the porous wick **3**, a vacuum is created in the head-space of the bottle **1**. The formation of a vacuum in the head-space of the bottle **1** decreases the rate that the liquid is wicked from the bottle **1** to the surface. Of course, this decrease in the wicking rate translates directly into a decrease in the release rate of the liquid to the ambient air.

In order to combat the formation of the vacuum in the head-space of the bottle **1**, many wick-based delivery systems contain a vent-hole in the vicinity of the head-space of the bottle **1**. These vent-holes prevent the formation of a vacuum in the head-space of the bottle **1** and, therefore, prevent the occurrence of a drop in the release rate of the liquid to the ambient air.

If the bottle **1** is overturned, either during shipping or, later, during handling of the bottle by the consumer, it is possible for the concentrated liquid in the bottle **1** to leak out of the vent-hole. Therefore, it is preferable to design a device that does not require a vent-hole.

We have found that if the pore size of the wick **3** is below a critical size, the vent-hole can be eliminated without sacrificing the release rate of the vaporizable liquid into the ambient air. Because the capillary force increases as the pore size of the wick **3** decreases, a wick **3** with very small porosity has a very strong capillary force. This strong capillary force allows the wick **3** to continue to be able to transport the liquid from the bottle **1** to the surface of the wick **3** even though a vacuum has formed in the head-space of the bottle **1**. In other words, a wick **3** with a very small pore size is able to overcome the vacuum effect that is present in the head-space of the bottle **1**.

The critical size of the wick **3** is determined by the surface tension of the liquid, the compatibility of the wick **3** and liquid (i.e., the contact angle), and the extent to which a vacuum is generated with the head-space of the bottle **1**. In particular, we have found that if the wick **3** is manufactured with a mean pore size that is below about four microns, the effects of a vacuum in the head-space of the bottle **1** can be greatly decreased. Specifically, we have found that it is most preferable that the mean pore size of the wick **3** be below about one micron. When the wick **3** has a mean pore size of below four microns, and preferably below one micron, we have found that the wick **3** is still able to effectively function to transport the liquid from the bottle **1** to the surface of the wick **3**.

When using a device of this invention, it is not necessary to provide a vent-hole in the upper part of the bottle **1**, or in the neck closure **2** because the vacuum effects are substantially decreased. By eliminating the vent-hole, the problem of spillage or leakage that occurs as a result of the existence of the vent-hole is also eliminated.

The mean pore size of the wick **3** can be determined by any standard test for determining porosity and pore size distribution. For example, mercury porosimetry is a method that gives information on porosity and pore size distribution for rigid wicks. It is based on the measurement of differential increments in the amount of mercury intruded into the wick **3** as a function of increasing applied pressure.

We have found that another advantage in using a wick **3** with a mean porosity of below about four microns, and preferably below about one micron, is that the lower porosity decreases the likelihood of the liquid spilling or leaking through the wick **3** itself. Since the upper surface of the wick **3** is exposed to the ambient air, if the bottle **1** is overturned, it is possible for liquid to leak out through a wick of conventional pore sizes. Using a smaller porosity wick **3** of this invention, however, decreases the ability of the liquid to travel through the wick **3** when the bottle **1** is overturned.

The wick **3** can be made of a variety of materials. It is preferable that the wick **3** be rigid enough to provide minimal contact area with the surface that the wick **3** comes in contact with. Polymeric wicks, for example, have been found to be effective for these purposes. In particular, wicks composed of ultra high molecular weight, high density polyethylene (HDPE) have been found to be suitable. Such wicks are generally comprised of blends of HDPE in particle form, and the blends are developed to meet the target pore characteristics of the wick **3**.

Preferably, the solubility parameter of the polymer used in the wick **3** is significantly different from that of any of the components contained in the liquid. This prevents the wick **3** from swelling (or other changes) that may lead to a change in the pore size and porosity of the wick **3**, which would consequently affect the release rate of the vaporizable liquid into the ambient air.

The wick **3** can also be made in a variety of shapes. Figure 1, for example, shows a cylindrical wick **3** with a narrower lower region. This change in shape of the wick **3** is not required. Instead, this variation in shape can be useful in that it both increases the amount of the surface area of the wick **3** that is exposed to the ambient air and aids in forming a tighter seal at the neck **5** area of the bottle **1**, thus helping to prevent spilling or leaking of the liquid from the bottle **1**. The above-described benefits of using a wick **3** with a mean pore size of below about four microns, and preferably below about one micron, can be obtained with wicks of many different shapes.

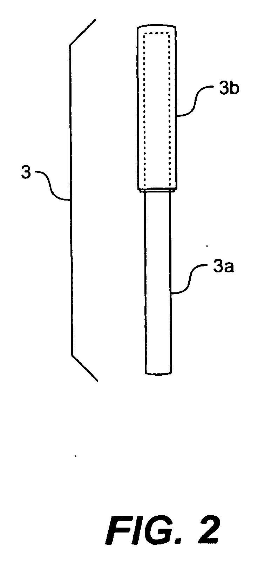

As shown in Figure 2, it is also possible to provide a wick **3** with an outer layer that is made up of a material with larger pore sizes. In Figure 2, the large pore outer section **3b** completely surrounds the exposed portion of the wick **3a**. The small pore size section 3a extends into the bottle **1** and is in contact with the liquid. In this manner, the smaller pores of the inner portion **3a** of the wick **3** allow the delivery system to be constructed without a vent-hole, while the larger pores of the outer portion **3b** provide a maximum release rate of the vaporizable liquid off the surface of the wick **3** that is exposed to the ambient air. It should be noted, however, that the large pore section **3b** need not completely surround the upper region of the small pore section 3a as shown in Figure 2 in order to provide the benefits of this invention.

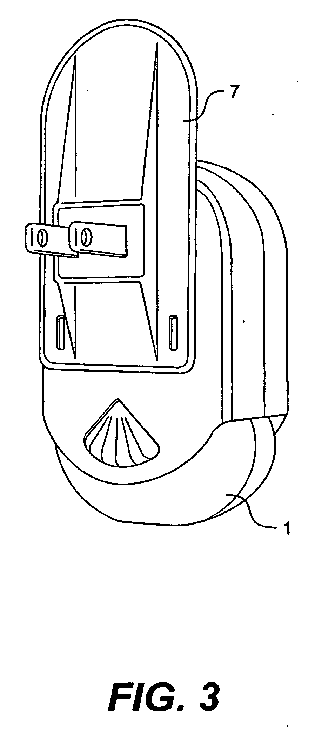

We also envision that our wick-based delivery system can be combined with an electric heater to facilitate the release of the vaporizable material into the ambient air. Figure 3 shows an example of the type of electric heater **7** that may be used for this purpose. U.S. Patent No. 5,647,053 describes such an electric plug-in heater and is incorporated herein by reference. Other means for facilitating the use of the wick-based delivery system of the invention are also envisioned. For example, the invention may also be combined with a battery powered fan. Although not required, it is preferable that the wick-based delivery system of the invention be combined with the electric plug-in heater or fan in a removable manner. For example, the wick-based delivery system of the invention may constructed so that the bottle 1 can be combined with an electric plug-in heater **7**, for example, in a snap-and-fit manner as shown in Figure 3.

### INDUSTRIAL APPLICABILITY

The present invention provides a device useful as a means to transport a liquid from a reservoir to a surface that is exposed to the ambient air. We envision that this device preferably can be used, for example, to dispense fragrances, insecticides, and any other vaporizable materials into the ambient air to freshen or deodorize the air or to exterminate airborne pests.

## Claims

1. A device comprising:
a container (1) for holding a liquid, the container being non-vented and having an opening (5) at a top surface of the container (1); and
a porous wick (3) having a predetermined mean pore size of less than one micron, the wick (3) extending through the opening (5) in the container (1) such that a lower region of the wick (3) will be in contact with the liquid to be held by the container (1) and an upper region of the wick is exposed to the ambient air, wherein the opening (5) in the container is substantially sealed by the wick (3);
**characterized in that** the device, further comprises:
a neck closure (2) having a hole, wherein the neck closure (2) fits tightly into the opening (5) of the container (1) and the wick (3) fits tightly into the hole of the neck closure (2), such that the opening of the container (1) is substantially sealed by the neck closure (2) and the wick (3); and
an outer layer (36) of porous material that surrounds at least a portion of the surface of the wick (3a) that is exposed to the ambient air, wherein the outer layer (36) has a predetermined mean pore size that is greater than that of the wick (3a).

2. The device of claim 1, wherein the wick (3) is comprised of high- density polyethylene

3. The device of claim 1, wherein the outer layer (36) completely surrounds the surface of the wick (3a) that is exposed to the ambient air.

4. The device of claim any preceding claim, further comprising a heater (7) arranged adjacent to the wick (3) for heating liquid drawn through the wick (3).

5. The device of claim 4, wherein the heater (7) is an electric plug-in heater.

6. The device according to any preceding claim, further including a battery powered fan.

7. The device according to any preceding claim further including the liquid held in said container.

8. The device according to any of claims 1-5 combined with an electric plug-in heater or fan, in a removable manner.

## Patentansprüche

1. Vorrichtung mit:
einem Behälter (1) zur Aufnahme einer Flüssigkeit, der keinen Druckausgleich enthält und eine Öffnung (5) an einer oben liegenden Oberfläche des Behälters (1) aufweist;
einem porösen Docht (3) vorbestimmter mittlerer Porengröße unter 1 µm, der durch die Öffnung (5) im Behälter (1) verläuft, so dass ein unterer Bereich des Dochts (3) in Berührung mit der Flüssigkeit im Behälter (1) steht und ein oberer Dochtbereich zur Umluft frei liegt, wobei die Öffnung (5) im Behälter vom Docht (3) im wesentlichen verschlossen wird;
**gekennzeichnet** weiterhin durch:
einen Halsverschluss (2) mit einem Loch, der fest in der Öffnung (5) des Behälters (1) sitzt, wobei der Docht (3) fest im Loch im Halsverschluss (2) sitzt derart, dass die Öffnung im Behälter (1) vom Halsverschluss (2) und Docht (3) im wesentlichen veschlossen wird; und
einer Außenschicht (36) aus porösem Material, die mindestens einen Teil (3a) der Oberfläche des Dochts, der zunächst zur Umluft frei liegt, umgibt, wobei die Außenschicht (36) eine vorbestimmte mittlere Porengröße hat, die größer ist als die des Dochts (3a).

2. Vorrichtung nach Anspruch 1, deren Docht (3) aus hochdichtem Polyethylen besteht.

3. Vorrichtung nach Anspruch 1, bei der die Außenschicht (36) die zunächst zur Umluft frei liegende Oberfläche (3a) des Dochts vollständig umgibt.

4. Vorrichtung nach einem der vorgehenden Ansprüche weiterhin mit einer Heizvorrichtung (7), die an den Docht (3) angrenzend angeordnet ist, um durch den Docht (3) gesaugte Flüssigkeit zu erwärmen.

5. Vorrichtung nach Anspruch 4, deren Heizvorrichtung (7) eine Elektrostecker-Heizvorrichtung ist.

6. Vorrichtung nach einem der vorgehenden Ansprüche weiterhin mit einem aus einer Batterie bzw. einem Akkumulator gespeisten Gebläse.

7. Vorrichtung nach einem der vorgehenden Ansprüche weiterhin mit einer im Behälter enthaltenen Flüssigkeit.

8. Vorrichtung nach einem der Ansprüche 1- 5 in Kombination mit einer Elektrostecker-Heizeinrichtung oder einem Gebläse, die trennbar ist.

## Revendications

1. Dispositif comprenant :
un conteneur (1) destiné à contenir un liquide, le conteneur étant non ventilé et ayant une ouverture (5) au niveau de la surface supérieure du conteneur (1) ; et
une mèche poreuse (3) possédant une dimension de pore moyenne prédéterminée de moins d'un micron, la mèche (3) s'étendant à travers l'ouverture (5) dans le conteneur (1) de sorte qu'une région inférieure de la mèche (3) sera en contact avec le liquide destiné à être contenu dans le conteneur (1) et une région supérieure de la mèche est exposée à l'air ambiant, où l'ouverture (5) dans le conteneur est sensiblement scellé par la mèche (3);
**caractérisé en ce que** le dispositif comprenant en outre :
une fermeture de col (2) possédant un orifice, où la fermeture de col (2) entre de manière serrée dans l'ouverture (5) du conteneur (1) et la mèche (3) entre de manière serrée dans l'orifice de la fermeture de col (2), de sorte que l'ouverture du conteneur (1) est sensiblement scellée par la fermeture de col (2) et la mèche (3) ; et
une couche extérieure (36) de matériau poreux qui entoure au moins une partie de la surface de la mèche (3a) qui est exposée à l'air ambiant, où la couche extérieure (36) possède une dimension de pore moyenne prédéterminée qui est supérieure à celle de la mèche (3a).

2. Dispositif de la revendication 1, dans lequel la mèche (3) est composé de polyéthylène à haute densité.

3. Dispositif de la revendication 1, dans lequel la couche extérieure (36) entoure complètement la surface de la mèche (3a) qui est exposée à l'air ambiant.

4. Dispositif de l'une quelconque des revendications précédentes, comprenant en outre un dispositif de chauffage agencé à côté de la mèche (3) pour chauffer le liquide attiré à travers la mèche (3).

5. Dispositif de la revendication 4, dans lequel le dispositif de chauffage (7) est un dispositif de chauffage enfichable électrique.

6. Dispositif selon l'une quelconque des revendications précédentes, comprenant en outre un ventilateur alimenté par piles.

7. Dispositif selon l'une quelconque des revendications précédentes comprenant en outre le liquide contenu dans ledit conteneur.

8. Dispositif selon l'une quelconque des revendications 1-5 combiné de manière amovible avec un chauffage enfichable électrique ou un ventilateur.
